# EUROPÄISCHE PATENTSCHRIFT

(11) **EP 1 025 207 B1**
(45) Veröffentlichungstag und Bekanntmachung des Hinweises auf die Patenterteilung: **07.09.2005**
(21) Anmeldenummer: 98955512.3
(22) Anmeldetag: 22.10.1998
(51) Int. Cl.: C12N 5/10, C12N 5/02

(54) **SUSPENSIONS-VERPACKUNGSZELLINIEN FÜR RETROVIRUSVEKTOREN**
SUSPENSION PACKING CELL LINES FOR RETROVIRUS VECTORS
LIGNEES DE CELLULES D'ENCAPSIDATION EN SUSPENSION POUR VECTEURS RETROVIRAUX

(30) Priorität: 25.10.1997 EP 97118583; 10.12.1997 EP 97121724
(43) Veröffentlichungstag der Anmeldung: 09.08.2000
(73) Patentinhaber: Roche Diagnostics GmbH, 68305 Mannheim (DE)
(72) Erfinder: SEEBER, Stefan, D-82377 Penzberg (DE); KOCH, Stefan, D-82377 Penzberg (DE); GUTJAHR, Thorsten, D-82377 Penzberg (DE); STEEGMANS, Ulrich, D-83670 Bad Heilbrunn (DE); RÜGER, Rüdiger, D-82386 Huglfing (DE)
(74) Vertreter: Schreiner, Siegfried
(86) Internationale Anmeldenummer: PCT/EP1998/006714
(87) Internationale Veröffentlichungsnummer: WO 1999/021967

(56) Entgegenhaltungen:
- EP-A- 0 792 931
- DE-A- 19 612 966
- US-A- 4 059 485
- F.-L. COSSET ET AL.: "High-titer packaging cells producing recombinant retroviruses resistant to human serum" JOURNAL OF VIROLOGY, Bd. 69, Nr. 12, Dezember 1995, Seiten 7430-7436, XP000569527 AMERICAN SOCIETY FOR MICROBIOLOGY US in der Anmeldung erwähnt
- DATABASE WPI Section Ch, Week 9604 Derwent Publications Ltd., London, GB; Class B04, AN 96-038261 XP002060320 & RU 1 347 448 C (A MED POLIOMYELITIS VIRUS INST), 10. April 1995

## Beschreibung

Gegenstand der Erfindung sind Verfahren zur Herstellung von serumfrei wachsenden Suspensions-Verpackungszellinien für retrovirale Vektoren.

Viren bestehen aus einem für die viralen Gene kodierenden Genom, sowie aus einem Viruscapsid, welches wie im Falle der Retroviren von einer Membranhülle umgeben sein kann.
Bei den Retroviren unterscheidet man die cis-Elemente 5'-LTR, 3'-LTR, Verpackungssequenz Psi, + und - Strang-Primerbindungsstelle von den trans-Elementen gag (Capsidproteine), pol (Reverse Transkriptase) und env (Hüllproteine). Die trans-Elemente können auf dem viralen Genom durch Fremdgene ersetzt werden; in diesem Fall ist das Virus jedoch auf sogenannte Helferviren angewiesen, welche entweder komplette, infektiöse Viren oder nichtinfektiöse Viren z.B. Viren ohne Verpackungssequenzen, sein können. Zellen, die solche nichtinfektiösen Helferviren und/oder Helfergene eines oder auch verschiedener Helferviren transient, episomal oder ins Genom integriert enthalten, werden als Verpackungszellinien bezeichnet. Verpackungszellinien setzen daher keine infektiösen, replikationskompetenten Viruspartikel frei. Stammen die Helfergene von verschiedenen Helferviren, so bezeichnet man die entstehenden Vektoren als chimäre Retrovirusvektoren; dieser Vorgang wird als Pseudotypisierung bezeichnet. Solche chimäre Verpackungssysteme werden üblicherweise verwendet, z.B. GP+env Am12 (Markowitz D.G. et al. in Trans. Assoc. Am. Physicians 101 (1988) 212 - 218) trägt die gag-pol Gene des ecotropen MoMuLV und das env-Gen des amphotropen 4070 Ampho-Virus. In Verpackungszellinien können sogar env-Gene mehrerer Viren (auch mit genetisch veränderten env-Genen) gemischt vorliegen.
Verpackungszellinien können die inkompletten viralen Helfergenome auch fragmentiert enthalten, wobei man dann von Verpackungszellinien mit gesplittetem Virusgenom spricht und welche eine erhöhte Sicherheitsvariante vor ungewollter Freisetzung infektiöser, replikationskompetenter Viren durch Rekombinationsereignisse darstellen.
Werden sie jedoch mit einem Retrovirusvektor transfiziert oder transduziert, der neben den cis-Elementen noch Fremdgene tragen kann, so werden diese Virusgenome verpackt und als infektiöse, doch nicht replikationskompetente Retrovirusvektoren freigesetzt. In diesem Fall spricht man von einer Retrovirusproduzentenzellinie.

Alle bisher hergestellten Retrovirusverpackungszellinien basieren auf adhärent, d.h. auf Oberflächen anhaftend wachsenden Zellen:

Von Markowitz, D.G. et al., werden in Trans. Assoc. Am. Physicians 101 (1988) 212 - 218 die Verpackungszellinien GP+E86 und GP+envAm12, basierend auf adhärenten NIH3T3-Zellen, beschrieben. Von Miller, A.D. et al. wird in Mol. and Cell. Biol. 6 (1986) 2895 - 2902 die Verpackungszellinie PA317, basierend auf adhärenten NIH3T3-Zellen, beschrieben. Von Miller, A.D. et al., J. Virol. 65 (1991) 2220 - 2224 wird die GALV-Verpackungszellinie PG13, basierend auf NIH3T3-Zellen, beschrieben. Danos, O, et al. beschreibt in PNAS USA 85 (1988) 6460 - 6464 die Verpackungszellinien ΨCre und ΨCrip, basierend auf NIH3T3-Zellen.

Rigg, R.J. et al. beschreibt in Virology 218 (1996) 290 - 295 die Verpackungszellinie Propack-A, welche auf einer humanen Zellinie basiert. Cosset, F.-L. et al. beschreiben in J. Virol. 69 (1995) 7430 - 7436 die Verpackungszellinien FLY A13 und FLY RD18, welche auf einer humanen Fibrosarcomalinie (HT10 80) basiert. In der WO 92/05266 werden Verpackungszellinien, basierend auf D 17 und anderen adhärenten Hundezellinien, beschrieben.

Auch die Mehrzahl der allgemein beschriebenen Zellinien wachsen adhärent. Adhärente Zellinien besitzen den Vorteil, daß sie einfach zu handhaben sind. So läßt es sich in adhärenten Zellen relativ einfach klonieren bzw. mit Viren, insbesondere Retroviren, arbeiten, d.h. eine klonale Selektion der Zellen nach Transfektion bzw. Transduktion ist direkt möglich, einzelne Kolonien lassen sich leicht erkennen, zählen und durch picken isolieren, tote Zellen lösen sich einfach ab und sind daher nicht mit lebenden zu verwechseln, zum Mediumwechsel muß einfach nur der Überstand abpipettiert und neues Medium zugegeben werden. Aussaat und Haltung der Zellen ist in der Regel unkritisch, da adhärente Zellen Kolonien von Zellen bilden, die sich wie im Gewebeverband gegenseitig stimulieren. Einzig beim Umsetzen der Zellen ist bei adhärenten Zellen eine zusätzliche Trypsin-Behandlung zum Ablösen notwendig, was aber in der Regel unkritisch ist.
In Suspension wachsende Zellen benötigen zur Klonierung und für Transduktionsexperimente ein anderes Methodenspektrum. So erfordert eine klonale Selektion das Arbeiten mit immobilisierenden Zusätzen wie Soft-Agars, eine als "Limited Dilution" bezeichnete Verdünnungsreihe der Zellen bis theoretisch nur mehr 1 Zellen pro Gefäß vorliegt, oder die Verwendung eines Oberflächenmarkers oder eines Fluoreszenzmarkers (wie GFP), welcher die direkte Selektion rekombinanter Zellen mittels eines FAC-Sorters ermöglicht. Lebende und sterbende Zellen lassen sich nur durch Färbungen unterscheiden. Ein Mediumwechsel erfordert immer einen Zentrifugationsschritt.
In Suspension wachsende Zellinien als Retrovirusverpackungszellinen sind bisher nicht bekannt. Es wurden auch bislang keine Versuche beschrieben, in denen etablierte, adhärent wachsende Verpackungszellinien in Suspension gebracht wurden.
Überraschenderweise zeigt sich, daß eine Umgewöhnung an Suspensionswachstum für adhärente Verpackungszellinien durchaus möglich ist und daß daraus resultierende Suspensionsverpackungszellinien in der Lage sind Viren mit hohen Titern zu produzieren. Des weiteren konnte gezeigt werden, daß es ebenso möglich ist aus klassischen Suspensionszellinien wie der humanen Erytholeukemia-Zellinie K562, der Ratten-Hepatomalinie N1-S1 und der Ratten-Lymphomalinie C58 durch Cotransfektion retroviraler Verpackungsgenplasmide mit retroviralen Vektorplasmiden in Suspension wachsende Retrovirusproduzentenzellinen herzustellen, d.h. daß diese Zellen Retrovirusvektorpartikel freisetzen, welche in der Lage sind andere Zellen zu transduzieren.

Die Herstellung von Suspensionsverpackungszellinien läuft technisch analog zu den für adhärente Zellen vielfach beschriebenen Verfahren durch Einbringen von Helfersequenzen (in einem Stück oder vorzugsweise gesplittet) die allein kein infektiöses Virus zu bilden in der Lage sind, bis auf den bereits erwähnten Unterschied, daß Suspensionszellen andere Klonierungs- und Kultivierungstechniken benötigen.
Ist eine Suspensionsverpackungszellinie hergestellt, kann sie durch Transduktion mit einem kompatiblen Retrovirusvektor (z.B. eine amphotrope Verpackungszelline mit einem aus einer ecotropen Verpackungszelline stammenden Vektor), vorzugsweise durch Transfektion mit einem Retroviursvektorplasmid, in eine Suspensionsproduzentenzellinie umgewandelt werden, welche infektöse doch replikationsinkompetente Retrovirusvektoren freisetzt.

Bisher sind keine vollständig in Suspension wachsende Säugerzellinien als Retrovirus-Verpackungszellinien bekannt. Es ist bisher auch kein Verfahren bekannt, in dem etablierte, adhärent wachsende Verpackungszellinien vollständig in Suspension gebracht wurden.

Aufgabe der Erfindung ist es, Verfahren zur Herstellung von serumfrei in Suspension wachsende Verpackungszellinien für retrovirale Vektoren sowie Verfahren zu ihrer Herstellung und Verwendung zur Verfügung zu stellen.

Gegenstand der Erfindung ist ein Verfahren zur Herstellung einer serumfrei in Suspension wachsende Verpackungszellinie, welche mit einem Retrovirusvektor transduziert ist.

Unter einer Verpackungszellinie ist eine Säugerzellinie zu verstehen, welche die für die Produktion von viralen Partikeln notwendigen Gene gag, pol und env (Helfergene), nicht jedoch funktionell aktive Verpackungssequenzen (Ψ) enthält. Eine solche Zellinie kann von sich aus keine Viruspartikel mit genomischer RNA bilden.

Unter einer funktionell aktiven Verpackungssequenz ist die Region eines Retrovirusgenoms zu verstehen, welche funktionell die Verpackung des RNA-Genoms bewirkt. Ihre Funktion kann durch vollständige oder teilweise Deletion oder durch Mutation desaktiviert werden.

Vorzugsweise wird erfindungsgemäß eine vollständig in Suspension wachsende (Zum Wachstum - auch über längere Zeit, z.B. Tage - wird keine unlösliche Matrix (Gefäßwand oder Microcarrier, an die die Zellen anhaften) benötigt.) Retrovirusverpackungszellinie aus einer adhärent wachsenden Retrovirusverpackungszellinie dadurch hergestellt, daß eine derartige adhärente Verpackungszellinie über einen Zeitraum von mindestens drei Monaten Schritt für Schritt an das Wachstum in Suspension adaptiert wird. Die Schritte beinhalten eine kontinuierliche Reduktion des FKS-Gehalts im Medium bis zur Serumfreiheit, wiederholte Behandlung der Zellen mit Trypsin und/oder DNAse, der Verwendung von Medium für Suspensionzellinien (beispielsweise DMF024A), vorzugsweise Gewebekulturflaschen für Suspensions- und Hybridomazellen (Sarstedt), kontinuierliches Schwenken der Kulturflaschen auf dem Schüttler sowie einer Zelldichte von zwischen 5 x 10⁴ bis 1 x 10⁶ Zellen/ml (es ist damit ein wesentlich häufigeres Splitten der Zellen als bei vergleichbarer adhärenter Kultur erforderlich).

Vorzugsweise wird die erfindungsgemäße, vollständig in Suspension wachsende Zellinie, welche mit einem Retrovirusvektorplasmid transfektiert oder mit einem Retrovirusvektor transduziert ist, dadurch hergestellt, daß eine Suspensionszellinie mit einem Retrovirusvektorplasmid transfektiert oder mit einem Retrovirusvektor transduziert wird, die transfektierten oder transduzierten Zellen über einen co-transfektierten oder co-transduzierten Oberflächenmarker durch limitierte Verdünnung und/oder den Zusatz immobilisierender Zusätze selektiert werden und die so selektierten erfindungsgemäßen Suspensionszellen isoliert werden. Vorzugsweise ist die Suspensionszellinie eine Retrovirusverpackungszellinie. Zur Herstellung einer in Suspension wachsenden Retrovirusverpackungszellinie wird vorzugweise eine adhärent wachsende Retrovirusverpackungszellinie, welche mit Nukleinsäuren, welche für Helfersequenzen codieren und mit Retrovirusvektorplasmid transfiziert oder Retrovirusvektor transduziert ist und in einem serumhaltigen Medium kultiviert wird, in eine in Suspension wachsende Zellinie überführt, durch kontinuierliche Reduktion des Serumgehalts im Medium bis zur im Wesentlichen Serumfreiheit, wiederholte Behandlung der Zellen mit Trypsin und DNAse zur Ablösung der Zellen vom Träger, wobei die Zelldichte in Suspension in einem Bereich zwischen 5 x 10⁴ bis 1 x 10⁶ Zellen/ml gehalten wird. Besonders bevorzugt wird dieses Verfahren bei einem längeren Zeitraum, vorzugsweise mindestens drei Monate, durchgeführt. In einer weiteren bevorzugten Ausführungsform werden die transfektierten oder transduzierten Zellen über einen co-transfektierten oder co-transduzierten Oberflächenmarker durch limitierte Verdünnung und/oder den Zusatz immobilisierender Zusätze selektiert.

Es hat sich überraschenderweise gezeigt, daß so behandelte vollständig in Suspension wachsende Verpackungszellinien trotz der massiven mehrmonatigen Langzeitmanipulation immer noch in der Lage sind, Retroviren zu produzieren, nachdem sie mit Retrovirusvektorplasmiden transfiziert bzw. mit Retrovirusvektoren transduziert wurden.

Ebenso überraschend ist es, daß derartige vollständig in Suspension wachsende Verpackungszellinien durch Transfektion mit Retrovirusvektorplasmiden bzw. Transduktion mit Retrovirusvektoren durch Vereinzelungsschritte, wie die limitierte Verdünnung, bei der die Zellen soweit verdünnt werden, bis praktisch nur noch Einzelzellen pro Gefäß in Suspension vorliegen oder durch die Verwendung immobilisierender Zusätze wie einem Overlay-Agar bzw. Methylcelluloses (Metho Cult H4100 / Stem Cell Technologies) oder einer Zellsortierung gegen einen Zelloberflächenmarker, kloniert werden können und daraus dann vollständig in Suspension wachsende Retrovirusproduzentenzellinien entstehen, die in selbem Maße in der Lage sind, Retrovirusvektoren zu produzieren wie adhärente Retrovirusproduzentenzellinien.

Derartig hergestellte Retrovirusproduzentenzellinien erlauben im Vergleich zu adhärenten Retrovirusprodzentenzellinien eine wesentlich einfachere großtechnische Kultivierung in Rührfermentern oder High-Density Dialysis Kulturverfahren.
Des weiteren unterscheiden sich die Gewinnungsmethoden der Vektorviren. Während bei adhärenten Retrovirusproduzentenzellinien die retrovirushaltigen Kulturüberstände lediglich abgenommen werden müssen, wobei ein Sterilfiltrationsschritt, wenngleich nicht zwingend notwendig , aus Sicherheitsgründen dennoch immer durchgeführt wird, müssen bei vollständig in Suspension wachsenden Retrovirusproduzentenzellinien die viel kleineren, leichteren Retrovirusvektorpartikel von den größeren, schwereren Produzentenzellinien durch einen Filtrations- und Zentrifugationsschritt oder mindestens 2 Filtrationsschritten abgetrennt werden.

Als Retrovirusvektorplasmide oder Retrovirusvektoren werden solche Retrovirusvektoren verwendet, welche ein therapeutisches Gen enthalten. Diese Vektoren enthalten keine Verpackungssequenzen wie gag, pol, env, um zu verhindern, daß replikationskompetente Retrovirusvektoren bei der Herstellung in der Suspensionsproduzentenzellinie freigesetzt werden.
Üblicherweise ist das therapeutisch relevante Gen zwischen 5'-LTR und 3'-LTR insertiert, wobei zweckmäßigerweise Selektionsgene wie das Neo- oder Hygromycin-Resistenzgen enthalten sind.

Unter einem replikationsinkompetenten (replikationsdefekten) Vektor ist ein Vektor zu verstehen, der keine retroviralen Genfunktionen (gag, env, pol) enthält und dadurch nicht selbständig Viruspartikel bilden kann. Üblicherweise enthält der Vektor jedoch eine packaging-Funktion (psi). Zur Bildung von infektiösen retroviralen Partikeln wird eine packaging-Zellinie (Verpackungszellinie), welche die Genfunktionen gag, env und pol stabil (als Episom oder ins Genom integriert) enthält, mit einem replikationsdefekten DNA-Vektor transduziert. Es werden RNA-Transkripte gebildet, die auf Grund der gag- und env-Funktionen in Viruspartikel verpackt werden. Diese Viruspartikel sind replikationsdefizient, weil das in ihnen enthaltene RNA-Genom keine retroviralen Genfunktionen trägt.

### Abkürzungen

- MoMuLV: Moloney murine leukemia virus
- FKS: Fötales Kälberserum
- ΔLNGFR: Low affinity nerve growth factor receptor, bei dem die intrazelluläre Domäne deletiert ist (vgl. WO 95/06723)
- SV40-P/E: Promotor/Enhancer Einheit von SV 40
- neoR: Neomycin Resistenz Gen
- pBR 322: E.coli Basisvektorplasmid
- T 25: Zellkulturflaschengröße
- CMV-P/E: Promotor/Enhancer Einheit von CMV (vgl. z.B. EP-B 0 173 177)
- TK-P: Herpes Simplex Thymidinkinase Promotor
- poly-A: Polyadenylierungsstelle
- hygR: hygromycin resistenz gen
- gpt: gpt Selektionsgen
- pUC19: Basisvektor
- pUC20: Basisvektor
- 5'LTR MoMuLV: 5'LTR aus MoMuLV
- cfu: colony forming units

Die folgenden Beispiele und Publikationen erläutern die Erfindung, deren Schutzumfang sich aus den Patentansprüchen ergibt, weiter. Die beschriebenen Verfahren sind als Beispiele zu verstehen, die auch noch nach Modifikationen den Gegenstand der Erfindung beschreiben.

### Beispiel 1: Adaption adhärenter Retrovirusverpackungszellinien an Wachstum in Suspension

Die adhärenten Verpackungszellinien FLY A13 (amphotropes env aus MoMuLV) und FLY RD18 (env aus cat endogenous virus RD114) (Cosset, F.-L. et al., J. Virol. 69 (1995) 7430 - 7436), welche beide auf der humanen Fibrosarcomalinie HT1080 basieren, wurden durch folgende Verfahrensschritte an Suspensionswachstum adaptiert:
a) Adaption der adhärenten Verpackungszellinien an Wachstum in Suspension:
   I. kontinuierliche und langsame FKS-Reduktion im Medium; Dauer ca. 5 Monate; 10% FKS ->5% ->2,5% ->2% ->1% ->0,5% ->Serum frei
   II. Verwendung eines Mediums für Suspensionszellinien
   III. Verwendung Kulturflaschen für Suspensionszellinien (Sarstedt)
   IV. kontinuierliches, leichtes Schwenken der Kulturflaschen auf Schüttler
   V. Die Haltung erfordert häufigeres Auflösen der entstandenen Zellklumpen durch Trypsin- und
      DNAse-Behandlung und häufigeres Splitten, damit sie in Suspension bleiben.
      Die Zelldichte ist immer ein kritischer Faktor (5 . 10⁴ - 1 . 10⁶ Zellen/ml), d.h. sie darf weder zu hoch noch zu dünn sein.
b) Bestimmung der Retrovirustiter transienter Kulturen
   Die in Suspension wachsenden Verpackungszellinien wurden bezüglich Produktionsverhalten von Retrovirusvektoren mit den entsprechenden adärent wachsenden Verpackungszellinien verglichen.
   Dazu wurden alle Zellinien mit dem Retrovirusvektorplasmid transfiziert und die Retrovirustiter der transienten Kulturen bestimmt.

### Material:

* Retroviraler Vektor pLΔNSN (6853 bp)
   [- 5'LTR MoMuLV - Ψ - ΔLNGFR - SV40-P/E - neoR - 3'LTR MoMuLV - pBR322-Plasmidanteil-]
* Transfektions-Reagenz: DOSPER; jeweils 20 µg DOSPER/Ansatz
   (Hersteller: Boehringer Mannheim GmbH, DE)
* Plasmid-DNA-Konz.: 5 µg DNA/Ansatz/Plasmid

### Methoden:

1. Tag: Zellen zur Transfektion einsäen.:
   - adhärente Zellen:: in 60 mm Petrischalen zu je 6x10⁵ Zellen/Schale
   - Suspensionszellen:: in Kulturflaschen für Suspensionszellen; (T25, Sarstedt) hochgestellt) zu je 3x10⁵ Zellen/ml; insgesamt 2 ml.
   Inkubation ü.N. bei37°C, 5% CO2, Suspensionszellen werden leicht geschüttelt.
2. Tag: Mediumwechsel (bei Suspensionszellen durch Zentrifugation und Resuspension), DNA/DOSPER-Mix hergestellt und auf Zellen auftropfen (je 100 µl DNA/DOSPER-Mix).
   Inkubation 5-6h, 37°C, 5% CO2. Anschließend Zugabe von Medium.
3. Tag: 24 h nach Transfektion Überstand abgenommen, zentrifugiert (nur bei Suspensionszellinien!), filtriert (0,45µm Filter) und auf NIH3T3 Zellen titriert (G418-Selektion).
   Titrationsergebnis ca. 10 - 14 Tage später.

### Titer:

| | | |
|---|---|---|
| adhärente Zellinien | FLY A13 | 8x105 cfu/ml 2x105 cfu/ml |
| | FLY RD18 | bisher nicht bestimmt |
| in Suspension gebrachte Zellinien | FLY A13 | 2,4x103 cfu/ml |
| | FLY RD18 | 1x102 cfu/ml |

### Ergebnis:

Die in Suspension gebrachten Verpackungszellinien sind prinzipiell in der Lage rekombinante
Retroviren zu produzieren.
Unterschiede im Titer lassen sich mit den unterschiedlichen Versuchsbedingungen bei der Transfektion und der Titerbestimmung zurückführen, z.B. unterschiedliche Transfektionseffizienzen in Suspensionszellen und adhärente Zellen und unterschiedliche Stabilität bzw. Infektiosität von Retroviren in FKS-freiem und FKS-haltigem Medium.

### Beispiel 2: Retrovirusproduktion nach transienter Tripeltransfektion der beiden Helferplasmide und dem Retrovirusvektorplasmid in Suspensionszellinien

Eine adhärente und mehrere in Suspension wachsende Zellinien wurden in transienten Tripeltransfektionsansätzen bezüglich der Fähigkeit infektiöse Retrovirusvektoren zu bilden verglichen.

### Verwendete Zellinien:

- adhärente Zellinie (Kontrolle): NIH3T3 (mouse fibrosarcoma)
- Suspensionszellinien:: K562 (Human erythroleukemia cell line)
N1-S1 (Rat Hepatoma)
C58 (Rat Lymphoma)

### Material:

**a) Helferplasmide:**
   * Gag-pol Expressions-Plasmid pGAPOGPT (10202 bp)
      [- CMV-P/E-**gag-pol**-polyA-SV40-P/E-gpt-polyA-pUC19-Plasmidanteil-]
   * ENV-Expressions-Plasmid pENVCHT (7933 bp)
      [-TK-P - hygR - polyA - CMV-P/E - **env (amphotrop)** - I poly A - pUC20-Plasmidanteil-]
**b) retrovirales Vektorplasmid:**
   * Retroviraler Vektor pLΔNSN (6853 bp)
      [- 5'LTR MoMuLV - Ψ - ΔLNGFR - SV40-P/E - neoR - 3'LTR MoMuLV - pBR322-Plasmidanteil-]
   * Transfektions-Reagenz: DOSPER (BM); jeweils 20 µg DOSPER/Ansatz
   * Plasmid-DNA-Konz.: 5 µg DNA/Ansatz/Plasmid

### Methoden:

1. Tag: Zellen zur Transfektion einsäen.:
   - adhärente Zellen (NIH3T3):: in 60 mm Petrischalen zu je 6x 105 Zellen/Schale
   - Suspensionszellen:: in T25 Kulturflaschen (für Suspensionszellen; hochgestellt) zu je 3x105 Zellen/ml; insgesamt 2 ml.
   Inkubation ü.N. bei 37°C, 5% CO2, Suspensionszellen werden leicht geschüttelt.
2. Tag: Mediumwechsel (bei Suspensionszellen durch Zentrifugation und Resuspension), DNA/DOSPER-Mix hergestellt und auf Zellen auftropfen (je 100 µl DNA/DOSPER-Mix).
   Inkubation 5-6h, 37°C, 5% CO2. Anschließend Zugabe von Medium.
3. Tag: 24 h nach Tripeltransfektion Überstand abgenommen, zentrifugiert (nur bei Suspensionszellinien!), filtriert (0,45µm Filter) und auf NIH3T3 Zellen titriert (G418 Selektion).
   Titrationsergebnis ca. 10 - 14 Tage später.

### Titrationsergebnis auf NIH3T3 (Anzahl G418-resistenter Kolonien):

NIH3T3 (adhärente Kontrollzellen): 3 Kolonien pro 1,4 ml Virusüberstand (Titer: 2 cfu/ml)
K562 : 6 Kolonien pro 0,8 ml Virusüberstand (Titer: 7,5 cfu/ml)
N1-S1: 6 Kolonien pro 2 ml Virusüberstand (Titer: 3 cfu/ml)
C58: 1 Kolonie pro 2 ml Virusüberstand (Titer: 0,5 cfu/ml)

### Referenzliste

Cosset, F.-L. et al., J. Virol. 69 (1995) 7430 - 7436
Danos, O. et al., PNAS USA 85 (1988) 6460 - 6464
Markowitz, D.G. et al., Trans. Assoc. Am. Physicians 101 (1988) 212 - 218
Miller, A.D. et al., J. Virol. 65 (1991) 2220 - 2224
Miller, A.D. et al., Mol. and Cell. Biol. 6 (1986) 2895- 2902
Rigg, R.J. et al., Virology 218 (1996) 290 - 295
WO 92/05266

## Patentansprüche

1. Verfahren zur Herstellung einer vollständig in Suspension wachsenden Verpackungszellinie, **dadurch gekennzeichnet, daß**
eine adhärent wachsende Verpackungszellinie, welche mit Nukleinsäuren, welche für Helfersequenzen codieren und mit einem Retrovirusvektorplasmid transfiziert oder Retrovirusvektor transduziert ist und in einem serumhaltigen Medium kultiviert wird, in eine in Suspension wachsende Zellinie überführt wird, durch
kontinuierliche Reduktion des Serumgehaltes im Medium bis zur Serumfreiheit, wiederholte Behandlung der Zellen mit Trypsin und/oder DNAse zur Ablösung der Zellen vom Träger, wobei die Zelldichte in Suspension in einem Bereich zwischen 5 x 10⁴ bis 1 x 10⁶ Zellen/ml gehalten wird.

2. Verfahren nach Anspruch 1, **dadurch gekennzeichnet, daß** das Verfahren über einen Zeitraum von mindestens drei Monaten durchgeführt wird.

3. Verfahren nach Anspruch 1 oder 2, **dadurch gekennzeichnet, daß** die transfektierten oder transduzierten Zellen über einen co-transfektierten oder co-transduzierten Oberflächenmarker durch limitierte Verdünnung und/oder den Zusatz immobilisierender Zusätze selektiert werden.

## Claims

1. Process for producing a packaging cell line that grows completely in suspension, **characterized in that**
an adherently growing packaging cell line which is transfected with nucleic acids that code for helper sequences and with a retrovirus vector plasmid or is transduced with a retrovirus vector and is cultured in a serum-containing medium, is converted into a cell line growing in suspension by
continuously reducing the content of serum in the medium until it is free of serum and repeatedly treating the cells with trypsin and/or DNAse to detach the cells from the substrate while keeping the cell density in suspension in a range between 5 x 10⁴ and 1 x 10⁶ cells/ml.

2. Process as claimed in claim 1, **characterized in that** the process is carried out for a period of at least three months.

3. Process as claimed in claim 1 or 2, **characterized in that** the transfected or transduced cells are selected by means of a co-transfected or co-transduced surface marker by limited dilution and/or the addition of immobilizing additives.

## Revendications

1. Procédé de fabrication d'une lignée de cellules d'encapsidation croissant complètement en suspension, **caractérisé en ce que**
une lignée de cellules d'encapsidation adhérente croissante, transfectée avec des acides nucléiques codant des séquences helper et avec un vecteur plasmidique rétroviral ou ayant subi une transduction par un vecteur rétroviral et cultivée dans un milieu contenant du sérum, est introduite dans une lignée cellulaire croissant en suspension, par
réduction continue de la teneur en sérum dans le milieu jusqu'à absence de sérum, traitement répété des cellules avec de la trypsine et/ou de l'ADNase pour séparation des cellules du porteur, la densité cellulaire dans la suspension étant maintenue dans un domaine entre 5x10⁴ et 1x10⁶ cellules/ml.

2. Procédé selon la revendication 1, **caractérisé en ce que** le procédé est réalisé en une période d'au moins trois mois.

3. Procédé selon la revendication 1 ou la revendication 2, **caractérisé en ce que** les cellules transfectées ou ayant subi une transduction sont sélectionnées par un marqueur de surface cotransfecté ou ayant subi une co-transduction par dilution limitée et/ou ajout d'additifs d'immobilisation.
